(19) Europäisches Patentamt
European Patent Office
Office européen des brevets

(11) Publication number: **0 003 901**
**B1**

(12) # EUROPEAN PATENT SPECIFICATION

(45) Date of publication of patent specification: **05.08.81**

(51) Int. Cl.³: **C 07 D 403/06,**
A 61 K 31/415

(21) Application number: **79300256.9**

(22) Date of filing: **20.02.79**

(54) **3-(Imidazol-1-ylalkyl)indoles as selective inhibitors of thromboxane synthetase pharmaceutical compositions thereof, and methods for preparing them.**

(30) Priority: **24.02.78 GB 740078**

(43) Date of publication of application:
**05.09.79 Bulletin 79/18**

(45) Publication of the grant of the European patent:
**05.08.81 Bulletin 81/31**

(84) Designated Contracting States:
**BE CH DE FR GB IT LU NL SE**

(56) References cited:
**EP - A - 0 000 355**
**CH - A - 551 572**
**DE - B - 1 156 813**
**US - A - 3 586 695**

**JOURNAL OF CHEMICAL SOCIETY (C), 1970, pages 1157—1161**

(73) Proprietor: **Pfizer Limited**
**Ramsgate Road**
**Sandwich Kent CT13 9NJ (GB)**

(73) Proprietor: **Pfizer Corporation**
**Calle 15 1/2 Avenida Santa Isabel**
**Colon (PA)**

(72) Inventor: **Cross, Peter Edward**
**21 Cherry Avenue**
**Canterbury Kent (GB)**
Inventor: **Dickinson, Roger Peter**
**6 Kingston Close River**
**Dover Kent (GB)**

(74) Representative: **Wood, David John et al,**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 003 901 B1

3-(Imidazol-1-ylalkyl)indoles as selective inhibitors of thromboxane synthetase, pharmaceutical compositions thereof, and methods for preparing them.

This invention relates to certain indole derivatives, specifically, to certain 3-(imidazol-1-ylalkyl)indoles, and to their use in selectively inhibiting the action of the thromboxane synthetase enzyme, i.e. without significantly inhibiting the action of the prostacyclin synthetase or cyclo-oxygenase enzymes. The compounds may thus be useful in, for example the treatment of of ischaemic disease, stroke, transient ischaemic attack, and migraine.

Thus, according to the invention there is provided a compound of the formula:

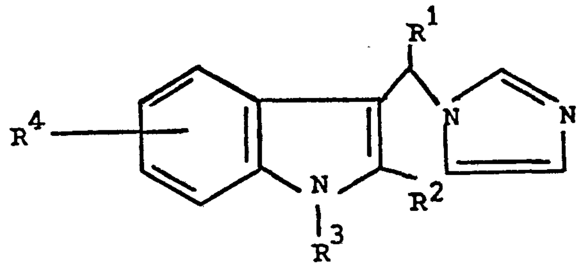

(I)

wherein

$R^1$ is hydrogen or lower alkyl;

$R^2$ is hydrogen, lower alkyl, lower cycloalkyl, adamantyl, or a phenyl or benzyl group in which the benzene ring is optionally mono- substituted with halogen, hydroxy, lower alkyl, lower alkoxy or trifluoromethyl;

$R^3$ is hydrogen, lower alkyl, allyl, 3-methyl-allyl, lower cycloalkyl-methyl, lower cycloalkyl-ethyl, lower alkoxy-lower alkyl, di(lower alkyl)amino-lower alkyl, lower alkanoyl, lower cycloalkylcarbonyl, or a benzyl or benzoyl group in which the benzene ring is optionally substituted as in $R^2$;

and $R^4$ is hydrogen, lower alkyl, lower alkoxy, halogen, hydroxy, trifluoromethyl, di(lower alkyl)amino, or a benzyloxy group in which the benzene ring is optionally substituted as in $R^2$;

*with the proviso* that at least one of $R^1$, $R^2$, $R^3$ and $R^4$ is other than hydrogen;

and the pharmaceutically acceptable acid addition salts thereof.

In this application, the term "lower" when applied to an alkyl, alkoxy or alkanoyl group means that the group contains not more than 4 carbon atoms, and when applied to a cycloalkyl group means that the group contains not more than 7 carbon atoms. The term "halogen" means fluorine, chlorine, bromine or iodine.

In addition the invention provides a pharmaceutical composition comprising a compound of the formula (I) (with proviso) or a pharmaceutically acceptable acid addition salt thereof, together with a pharmaceutically acceptable diluent or carrier.

The preparation of the compound in which $R^1$, $R^2$, $R^3$ and $R^4$ are all hydrogen, i.e. 3-(imidazol-1-ylmethyl)indole, has been described in C. R. Acad. Sci., Paris, Ser. C 1968, *266* (15), 1168—80 and J. Chem. Soc. (C), 1970, 1157—1161, but no previous medical or any other use has been proposed for it.

Imidazolinyl indoles of the formula:—

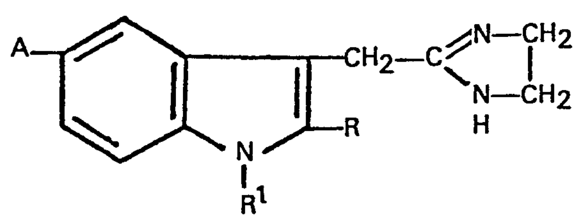

are disclosed in U.S. Patent No. 3,586,695, wherein A=halogen or alkyl or alkoxy of 1 to 3 carbon atoms, and R and $R^1$ are each independently hydrogen or alkyl of up to 3 carbon atoms. The compounds are said to be particularly useful as antidepressants, and may also be employed as appetite suppresants.

The invention yet further provides a compound of the formula (I) but in which $R^1$, $R^2$, $R^3$ and $R^4$ are hydrogen, or a pharmaceutically acceptable acid addition salt thereof, for use in treating an animal, including a human being, to inhibit the action of the thromboxane synthetase enzyme without significantly inhibiting the action of the prostacyclin synthetase or cyclo-oxygenase enzymes.

The invention also includes a pharmaceutical composition in unit dosage form (as hereinafter defined) comprising a compound of the formula (I), without proviso, or a pharmaceutically acceptable acid addition salt thereof, or a pharmaceutically acceptable precursor therefor, together with a pharmaceutically acceptable diluent or carrier.

By the term "unit dosage form" as used herein is meant a physically discrete unit containing an individual quantity of the active component in association with a pharmaceutically acceptable diluent or carrier, the quantity of active component being such that at least one unit or severable fraction of a unit

is required for a single therapeutic administration. In the case of severable units, such as scored tablets, at least one severable fraction such as a $\frac{1}{2}$ or $\frac{1}{4}$ of the unit may be all that is required for a single therapeutic administration. It will be appreciated that the term "unit dosage form" does not include mere solutions except when the solutions are packaged in ingestible containers, e.g. soft capsules, or have been prepared so as to be suitable for parenteral administration, e.g. in vials of solution suitable for parenteral injection.

Pharmaceutically acceptable acid addition salts of the compounds of the invention are salts with acids containing pharmaceutically acceptable anions, e.g. the hydrochloride, hydrobromide, sulphate or bisulphate, phosphate or acid phosphate, acetate, maleate, fumerate, lactate, tartrate, citrate, gluconate, succinate and p-toluene sulphonate salts.

Preferred compounds of the invention are those in which (a) $R^1$ is hydrogen or methyl; (b) $R^3$ is hydrogen and $R^2$ is hydrogen, isopropyl, cyclopropyl, cyclohexyl, benzyl, o- or p-tolyl or o-chlorophenyl; (c) $R^2$ is hydrogen and $R^3$ is methyl, ethyl, n-propyl, allyl, cyclopropylmethyl, acetyl or p-methyl- or p-methoxy-benzoyl; and (d) $R^4$ is hydrogen.

Where the compounds of the invention contain an asymmetric centre, the invention includes the racemic mixtures and the separated D- and L- optically active isomeric forms. Such forms should be obtainable by conventional methods, e.g. by fractional crystallisation of a salt with a suitable optically active acid.

The compounds of the invention may be prepared by a number of routes, including the following:—

(1) The compounds of the invention in which $R^3$ is hydrogen may be prepared by reacting an indole of the formula:

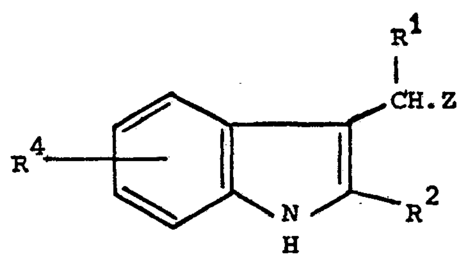

(II)

wherein $R^1$, $R^2$ and $R^4$ are as defined above and Z is a leaving group as defined hereinafter with imidazole.

Z is an $-N(C_1-C_4\text{ alkyl})_2$, $-\overset{\oplus}{N}(C_1-C_4\text{ alkyl})_3$, $-Cl$, $-Br$, $-OSO_2(C_1-C_4\text{ alkyl})$ or $-OSO_2$(phenyl, tolyl or *p*-methoxyphenyl) group.

Z is most preferably $-N(CH_3)_2$.

In a typical procedure, the compound of the formula (II) and imidazole are refluxed together in a suitable solvent, e.g. xylene, for from 1 to 3 hours. The solution is then cooled to crystallise the desired product out of solution. If necessary, petroleum ether (b.p. 60—80°C) may be added to induce crystallisation. The product may then be filtered off and recrystallised from a suitable solvent.

The starting materials of the formula (II) are either known compounds or may be prepared by procedures analogous to those of the prior art, e.g. as follows:—

(i)

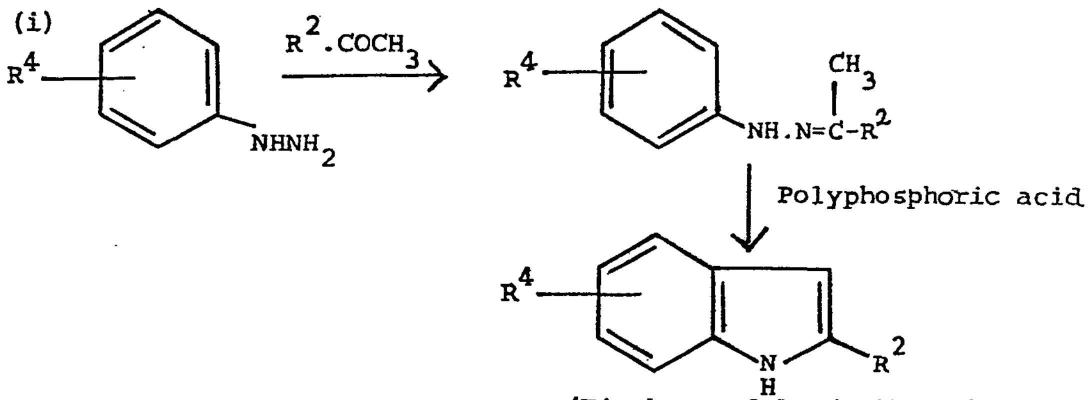


(Fischer Indole Synthesis)

or 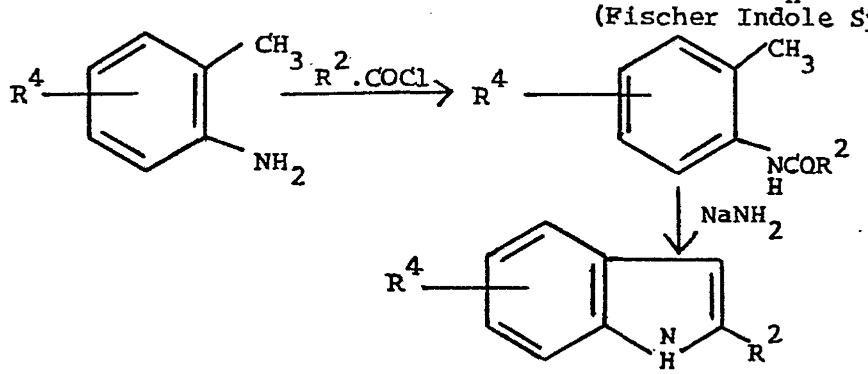


(see e.g. J. Org. Chem., *33*, 4285).

and then e.g. (ii)

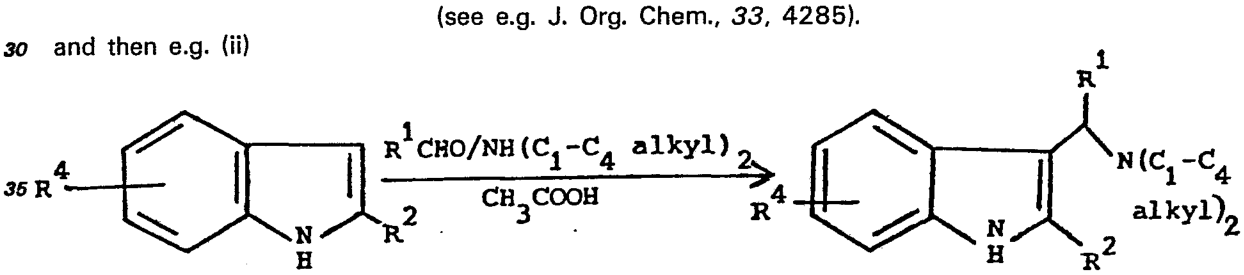


(Mannich reaction)

Again, compounds in which Z is a leaving group other than $—N(C_1—C_4\ alkyl)_2$ may be prepared by conventional procedures. For example, compounds in which Z is $—\overset{+}{N}(C_1—C_4\ alkyl)_3$ are generally preparable by alkylation of the corresponding dialkylamino derivative with the appropriate alkyl iodide; and the compounds in which Z is Cl or Br are generally preparable by halogenation of the corresponding hydroxy compounds (which are either known compounds or are preparable by conventional methods). The compounds in which Z is $—OSO_2(C_1—C_4$ alkyl) or $—OSO_2$(phenyl, tolyl or p-methoxyphenyl) are also generally preparable from the corresponding hydroxy compounds, viz. by reacting them with the appropriate alkyl or aryl sulfonyl chloride in the presence of an organic base.

(2) The compounds of the invention in which $R^3$ is other than hydrogen may be prepared by reacting the corresponding compound of the formula (I) in which $R^3$ is hydrogen with an appropriate alkylating or acylating agent in the presence of a base such as sodium hydride which forms the anion:

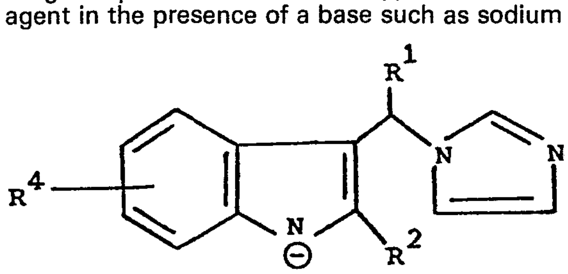

In a typical procedure the appropriate compound of the formula (I) in which $R^3$ is hydrogen is dissolved in a suitable solvent, e.g. dry dimethylformamide, and sodium hydride is then added carefully. The appropriate alkylating or acylating agent is then carefully added, and the resulting solution stirred at room temperature for up to 24 hours. The reaction mixture may then be poured into water, and the resulting mixture extracted with a suitable solvent, e.g. ethyl acetate, and the organic phase washed

with water, dried and evaporated to give the desired product, which if necessary may be recrystallised from a suitable solvent.

(3) Certain compounds of the invention may be prepared by conventional means from other compounds of the invention, e.g. compounds in which $R^2$ or $R^3$ contains a benzene ring substituted with a hydroxy group, or in which $R^4$ is a hydroxy group, can be prepared by demethylation or debenzylation of the corresponding methoxy and benzyloxy compounds.

(4) The pharmaceutically acceptable acid addition salts of the compounds of the invention may be prepared by conventional procedures, e.g. by reacting the free base in a suitable solvent, e.g. ethanol, with a solution of the appropriate acid in a suitable solvent, e.g. ether, thus generally precipitating the desired salt.

The compounds of the invention inhibit the action of the thromboxane synthetase enzyme, but do not significantly inhibit the action of the prostacyclin synthetase or cyclo-oxygenase enzymes.

Thus the compounds are useful in conditions characterised by an imbalance of prostacyclin/thromboxane $A_2$, which may for example include migraine, ischaemic heart disease, stroke and transient ischaemic attack, as explained below.

Research work has established that in most tissues the major product of the arachidonic acid metabolism is either of two unstable substances, thromboxane $A_2$ ($TxA_2$) or prostacyclin ($PGI_2$). (Proc. Nat. Acad. Sci. U.S.A., 1975, *72,* 2994; Nature, 1976, *263,* 663; Prostaglandins, 1976, *12,* 897). In most cases the prostaglandins $PGE_2$, $PGE_{2\alpha}$ and $PGD_2$ are comparatively minor by-products in this biosynthetic pathway. The discovery of thromboxane $A_2$ and prostacyclin has significantly increased our understanding of vascular homeostasis; prostacyclin for instance is a powerful vasodilator and inhibitor of platelet aggregation, and in this last respect is the most potent endogenous substance so far discovered.

The prostacyclin synthetase enzyme is located in the endothelial layer of the vasculature, and is fed by endoperoxides released by blood platelets coming into contact with the vessel wall. The prostacyclin thus produced is important for prevention of platelet deposition on vessel walls. (Prostaglandins, 1976, *12,* 685; Science, 1976, 17; Nature, 1978, *273,* 765).

Thromboxane $A_2$ is synthetised by the thromboxane synthetase enzyme which is located in, for example, the blood platelets. Thromboxane $A_2$ is a powerful vasoconstrictor and pro-aggregatory substance. As such its actions are in direct opposition to those of prostacyclin. If, for any reason, prostacyclin formation by the vasculature is impaired, then the endoperoxides produced by platelets coming into contact with the vessel wall are converted into thromboxane, but are not converted effectively into prostacyclin (Lancet, 1977, 18; Prostaglandins, 1978, *13,* 3). Alteration of the prostacyclin/thromboxane balance in favour of the latter substance could result in platelet aggregation, vasospasm (Lancet, 1977, 479; Science 1976, 1135; Amer. J. Cardiology, 1978, *41*, 787) and an increased susceptibility to atherothrombosis (Lancet (i) 1977, 1216). It is also known that in experimental atherosclerosis prostacyclin generation is suppressed and thromboxane $A_2$ production is enhanced (Prostaglandins, 1977, *14,* 1025 and 1035).

Thus thromboxane $A_2$ has been implicated as the causative agent in variant angina, myocardial infarction, sudden cardiac death and stroke (Thromb. Haemostasis, 1977, *38,* 132). Studies in rabbits have shown that ECG changes typical of these conditions were produced when freshly prepared thromboxane $A_2$ was injected directly into the animal's heart (Biochem. Aspects of Prostaglandins and Thromboxanes, Editors, N. Kharasch and J. Fried, Academic Press 1977 page 189). This technique is considered to represent a unique animal model of the heart attacks of coronary patients and has been used to show that administration of a compound believed to antagonise the effects of thromboxane $A_2$ protects the rabbits from the adverse consequences of thromboxane $A_2$ injection.

Another area where a $PGI_2/TxA_2$ imbalance is considered to be a contributory factor is that of migraine. The migraine headache is associated with changes in intra and extra-cerebral blood flow, in particular a pre-headache reduction of cerebral blood flow followed by dilatation in both vascular areas during the headache phase.

Prior to the development of the headache, blood levels of 5-hydroxytryptamine are elevated, and this suggests the occurrence of *in vivo* aggregation and release of the amine from the platelet stores. It is known that the blood platelets of migraine patients are more prone to aggregate than are those of normal individuals (J.clin.Pathol., 1971, *24,* 250; J.Headache, 1977, *17,* 101).

Furthermore it has now been postulated that not only is an abnormality of platelet function a major factor in the pathogenesis of migraine attacks but it is in fact their prime cause (Lancet (i), 1978, 501). Thus a drug that selectively modifies platelet function to inhibit thromboxane $A_2$ formation could be of considerable benefit in migraine therapy.

Aspirin and most other non-steroidal anti-inflammatory drugs inhibit the cyclo-oxygenase enzyme. The effect of this is to shut down the production of the $PGG_2/H_2$ endoperoxides and by so doing to reduce both the prostacyclin and thromboxane $A_2$ levels. Aspirin and aspirin-like drugs have been evaluated clinically for prevention of stroke and heart attack (New England J.Med., 1978, *299,* 53; B.M.J., 1978, 1188; stroke, 1977, *8* 301).

Although some encouraging results have been obtained with these drugs, a compound which

specifically inhibits thromboxane $A_2$ formation leaving the biosynthesis of prostacyclin unimpaired would be more valuable in these clinical conditions (Lancet, (ii), 1978, 780).

The effect of the compounds of the formula (I) on the thromboxane synthetase enzyme, and the prostacyclin synthetase and cyclo-oxygenase enzymes has been measured by the following *in vitro* enzyme assays:—

1. *Cyclo-oxygenase*

Ram seminal vesicle microsomes (Biochemistry, 1971, *10*, 2372) are incubated with arachidonic acid (100$\mu$M: 1 min.: 22°) to produce $PHG_2$ and aliquots of the reaction mixture injected into a stream of Krebs-bicarbonate at 37°C (containing a mixture of antagonists (Nature, 1978, *218*, 1135) and indomethacin (Brit. J. Pharmacol., 1972, *45*, 451)) which is superfusing a spirally-cut rabbit aorta strip (Nature, 1969, *223*, 29). The ability of a compound to inhibit the enzyme is measured by comparing the increases in isometric tension produced by $PGH_2$ in the absence of the test compound, and following pre-incubation of the enzyme with the test compound for 5 minutes.

2. *Prostacyclin ($PGI_2$) Synthetase*

Pig aorta microsomes (Nature, 1976, *263*, 663) are incubated (30 sec.; 22°C) with $PGH_2$ produced as in 1) and aliquots bio-assayed as in 1. $PGI_2$ production is assessed indirectly by measuring the decrease in $PGH_2$-induced tension ($PGI_2$ itself does not contract the aorta). This decrease can be prevented completely by pre-incubation of the enzyme with the selective $PGI_2$ synthetase inhibitor, 15-hydroperoxy-arachidonic acid (Prostaglandins, 1976, *12*, 715). The test compound is then pre-incubated with the enzyme for 5 minutes, and its ability to prevent the decrease in tension is measured.

3. *Thromboxane $A_2$ ($TxA_2$) Synthetase*

Indomethacin pretreated human platelet microsomes (Science 1976, *193*, 163) are incubated (2 min.: 0°C) with $PGH_2$ (produced as in 1) and aliquots of the reaction mixture superfused over two rabbit aorta spirals which are separated by a delay coil (2 min.). The latter is required to allow the selective decay of the more unstable thromboxane $A_2$ (Proc. Nat. Acad. Sci., 1975, *72*, 2994) thereby enabling the separate measurement of increased isometric tension due to the $TxA_2$ formed and the $PGH_2$ remaining.

The test compound is pre-incubated with the enzyme for 5 minutes, and its ability to inhibit the thromboxane synthetase enzyme is measured as its reduction of the $TxA_2$ component of the isometric tension.

Compounds of the invention tested in this way have been shown to be capable of selectively inhibiting the thromboxane synthetase enzyme.

In addition to the above an *in vito* assay for measuring the inhibition of human blood platelet aggregation has been described and this may be predictive of anti-thrombotic efficacy clinically (Lancet (ii), 1974, 1223; J. Exp. Med., 1967, *126*, 171). Both the clinically effective agents aspirin and sulphinpyrazone show inhibitory activity *in vitro* against a variety of aggregating agents in this test.

A number of *in vivo* tests in animals have also been described for evaluating potential anti-thrombotic drugs. Intravenous injection of arachidonic acid causes death in rabbits by causing platelet clumping and embolisation in the lungs. Again both the clinically effective aspirin (Agents and Actions, 1977, 1, 481) and sulphinpyrazone (Pharmacology, 1976, *14*, 522) protect the rabbit from the lethal effect of the injection. Sulphinpyrazone has also been shown to prevent the aggregation of platelets in an extra corporeal loop of the abdominal aorta of rats *in vivo* (Thromb. Diathes. Haem., 1973, *30*, 138).

The compounds of the invention are expected to be effective inhibitors of human blood platelet aggregation in the above *in vitro* assay, to protect rabbits against the lethal effect of arachidonic acid injection, and to prevent aggregation of platelets in the rat aorta.

The compounds may be administered orally in the form of tablets or capsules containing a unit dose of the compound together with such excipients as maize starch, calcium carbonate, dicalcium phosphate, alginic acid, lactose, magnesium stearate, "Primogel" (Trade Mark) or talc. The tablets are typically prepared by granulating the ingredients together and compressing the resulting mixture to tablets of the desired size. The capsules are typically prepared by granulating the ingredients together and filling them into hard gelatine capsules of the appropriate size to contain the ingredients.

The compounds may also be administered parenterally, for example by intramuscular, intravenous or subcutaneous injection. For parenteral administration, they are best used in the form of a sterile aqueous solution which may contain other solutes such as tonic and pH adjusters. The compounds may e.g. be added to distilled water and the pH adjusted to 3—6 using an acid such as citric, lactic or hydrochloric. Sufficient solutes such as dextrose or saline may be added to render the solution isotonic.

The resulting solution may then be sterilised according to the method of B.P. 1973 by filtration through a bacteria-proof filter under aseptic conditions into sterile containers so as to comply with the test for sterility of Appendix 121, B.P. 1973. Suitable containers are for example sterile glass vials of an appropriate size to contain the desired volume of solution, which volume will typically contain a unit dose of the compound of the formula (I). The compounds of the invention may also be administered by the infusion of a parenteral formulation as described above into a vein.

For oral administration to human patients, it is expected that the daily dosage level of a compound of the invention will be from 0.1 to 20 mg/kg. per day for a typical adult patient (70 kg.). For parenteral administration, it is expected that the daily dosage level of a compound of the formula (I) will be from

0.01—0.5 mg/kg. per day, for a typical adult patient. Thus tablets or capsules can generally be expected to contain from 5 to 150 mg of the active compound for administration orally up to 3 times a day. Dosage units for parenteral administration can be expected to contain from 0.5—35 mg of the active compound. A typical vial could be a 10 ml. vial containing 5 mg of the active compound in 6—10 ml of solution.

It should of course be appreciated that the physician in any event will determine the actual dosage which will be most suitable for the individual and it will vary with the age, weight and response of the patient. The above dosages are exemplary of the average host. There may of course be individual cases where higher or lower dosage ranges are merited.

The following Examples, in which all temperatures are given in °C, illustrate the invention. Gramine is the trivial name for 3-(dimethylaminomethyl)indole.

Example 1

Preparation of 3-(Imidazol-1-ylmethyl)-2-isopropylindole

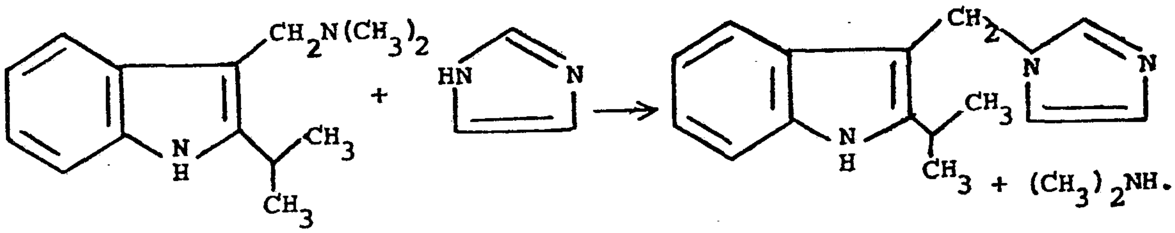

2-*Iso*-propyl gramine (1.7 g) and imidazole (0.6 g) were heated at reflux in xylene (50 ml) for a period of 1 hour. The solution was then cooled and 60—80° petroleum ether was added until a faint cloudiness appeared. The solution was then allowed to stand at room temperature (20°C) whereupon the product, 3-(imidazol-1-ylmethyl)-2-*iso*propylindole, crystallised out and was filtered off and recrystallised from industrial methylated spirits (yield: 0.5 g; m.p. 214—216°).

*Analysis %:—*

| | | | |
|---|---|---|---|
| Found: | C, 75.3; | H, 7.2; | N, 17.8 |
| Calculated for $C_{15}H_{17}N_3$: | C, 75.3; | H, 7.15; | N, 17.55. |

Examples 2—25

The following compounds were prepared similarly to Example 1, starting from an appropriately substituted gramine and imidazole:

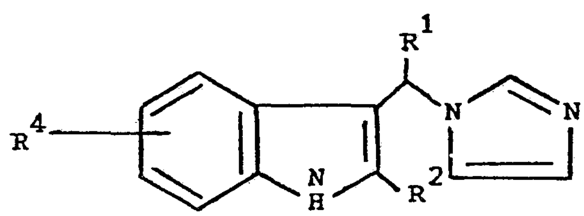

| Example No. | $R^1$ | $R^2$ | $R^4$ | Recrystallisation Solvent | m.p. (°C) | Analysis % (Calculated in brackets) C | H | N |
|---|---|---|---|---|---|---|---|---|
| 2 | H | $CH_3$ | H | ethyl acetate | 145–146 | 74.05 (73.9 | 6.15 6.2 | 20.1 19.9) |
| 3 | H | $C_6H_5$ | H | aqueous ethanol | 184–185 | 79.0 (79.1 | 5.6 5.5 | 15.4 15.4) |
| 4 | H | H | 5-$CH_3O$– | ethyl acetate/ petroleum ether (b.p. 60–80°) | 156–158 | 68.6 (68.7 | 5.8 5.8 | 18.65 18.5) |
| 5 | H | $CH_3$ | 5-$CH_3O$– | ethyl acetate/ petroleum ether (b.p. 60–80°) | 147–149 | 69.3 (69.7 | 6.4 6.3 | 17.6 17.4) |
| 6 | H | $(CH_3)_3C$ | H | aqueous ethanol | 215–216 | 76.0 (75.85 | 7.5 7.55 | 16.8 16.6) |
| 7 | $CH_3$ | H | H | aqueous ethanol | 172–173 | 73.6 (73.9 | 6.3 6.2 | 19.6 19.9) |
| 8 | H | H | 5-F | ethyl acetate | 168–170 | 66.89 (66.96 | 4.63 4.68 | 19.71 19.53) |
| 9 | H | H | 5-Br | methyl ethyl ketone | 198–201 | 52.34 (52.19 | 3.56 3.65 | 15.57 15.22) |

| Example No. | $R^1$ | $R^2$ | $R^4$ | Recrystallisation Solvent | m.p. (°C) | Analysis % (Calculated in brackets) C | H | N |
|---|---|---|---|---|---|---|---|---|
| 10 | H | H | 7-$CH_3$ | methyl ethyl ketone/petroleum ether (b.p. 60–80°) | 201–203 | 73.68<br>(73.90 | 6.25<br>6.20 | 19.83<br>19.89) |
| 11 | H | H | 5-$CH_3$ | methyl ethyl ketone/petroleum ether (b.p. 60–80°) | 189–191 | 73.55<br>(73.90 | 6.21<br>6.20 | 19.94<br>19.89) |
| 12 | H | cyclohexyl | H | industrial methylated spirits | 224–226 | 77.10<br>(77.38 | 7.59<br>7.58 | 15.05<br>15.04) |
| 13 | H | cyclopropyl | H | Toluene | 174–176 | 76.42<br>(75.92 | 6.48<br>6.37 | 17.52<br>17.71) |
| 14 | H | adamantyl | H | Ethyl Acetate/Trace Methanol | 266–268 | 79.04<br>(79.72 | 7.75<br>7.60 | 11.96<br>12.68) |
| 15 | H | 2-methylphenyl | H | Ethyl Acetate/Petrol (b.p. 80–100°) | 156–158 | 79.22<br>(79.41 | 6.19<br>5.96 | 14.53<br>14.62) |
| 16 | H | 2-methylphenyl | 5-$OCH_3$ | Isopropanol/Petrol (b.p. 60–80°) | 145–147 | 76.01<br>(75.68 | 6.09<br>6.03 | 13.33<br>13.24) |
| 17 | H | 4-methylphenyl | H | Ethyl Acetate | 194–196 | 78.92<br>(79.41 | 6.01<br>5.96 | 14.46<br>14.62) |

| Example No. | $R^1$ | $R^2$ | $R^4$ | Recrystallisation Solvent | m.p. (°C) | Analysis % (Calculated in brackets) C | H | N |
|---|---|---|---|---|---|---|---|---|
| 18 | H | 2-methoxyphenyl | H | Methanol | 199–201 | 75.44 (75.22 | 5.77 5.65 | 13.80 13.85) |
| 19 | H | 4-methoxyphenyl | H | Ethyl Acetate | 198–199 | 74.71 (75.22 | 5.62 5.65 | 13.51 13.85) |
| 20 | H | 2-chlorophenyl | H | Methanol | 235–237 | 70.13 (70.24 | 4.59 4.58 | 13.60 13.65) |
| 21 | H | 4-chlorophenyl | H | Methanol | 208–209 | 69.79 (70.24 | 4.66 4.58 | 13.78 13.65) |
| 22 | H | benzyl | H | Ethanol /Water | 159–160 | 79.24 (79.41 | 6.01 5.96 | 14.89 14.62) |
| 23 | H | H | 5-$N(CH_3)_2$ | Ethyl Acetate/Trace Methanol | 151–152 | 69.79 (69.97 | 6.78 6.71 | 23.01 23.32) |
| 24 | H | H | 6-$CF_3$ | Ethyl Acetate/Petrol (b.p. 40–60°) | 171 | 58.87 (58.87 | 3.80 3.80 | 15.83 15.84) |
| 25 | H | H | 5-benzyloxy | 2-Butanone/Petrol (b.p. 60–80°) | 194–196 | 75.07 (75.22 | 5.69 5.65 | 13.54 13.85) |

Example 26

Preparation of 1-benzyl-3-(imidazol-1-ylmethyl)-indole hydrochloride

3-(Imidazol-1-ylmethyl)indole, (1.97 g; 0.01 m) was dissolved in dry dimethylformamide (10 ml) and sodium hydride (0.32 g, 80% dispersion in oil) was added in portions with cooling. The solution was stirred at room temperature for 30 minutes and then benzyl bromide (1.71 g, 0.01 m) was added dropwise over 2 minutes with cooling. The solution was then stirred at room temperature (20°) for 2 hours, and poured into water to give an oil which solidified. The solidified oil/water mixture was extracted with ethyl acetate, and the resulting organic phase was washed well with water, and dried ($Na_2SO_4$). Evaporation of the organic phase gave an oil which solidified on standing. The solid was dissolved in a small volume of ethanol and an excess of ethereal hydrogen chloride was added to precipitate the hydrochloride salt of 1-benzyl-3-(imidazol-1-ylmethyl) indole as an oil, which solidified on standing: the solid was recrystallised twice from methyl ethyl ketone/ether to give 1.1 g of the purified product, m.p. 126—129°.

*Analysis %:—*

| | | | |
|---|---|---|---|
| Found: | C, 70.14; | H, 5.53; | N, 12.72 |
| Calculated for $C_{19}H_{17}N_3{\cdot}HCl$: | C, 70.47; | H, 5.60; | N, 12.98. |

Examples 27—45

The following compounds were prepared similarly to Example 26 starting from 3-(imidazol-1-ylmethyl)indole, appropriately substituted if necessary, and using an appropriate alkylating or acylating agent $R^3X$. In all cases the crude product was purified by chromatography on silica gel. Elution with petrol removed mineral oil and the pure product was eluted with chloroform, except in the case of Example 38 where it was eluted with 9/1 chloroform/methanol.

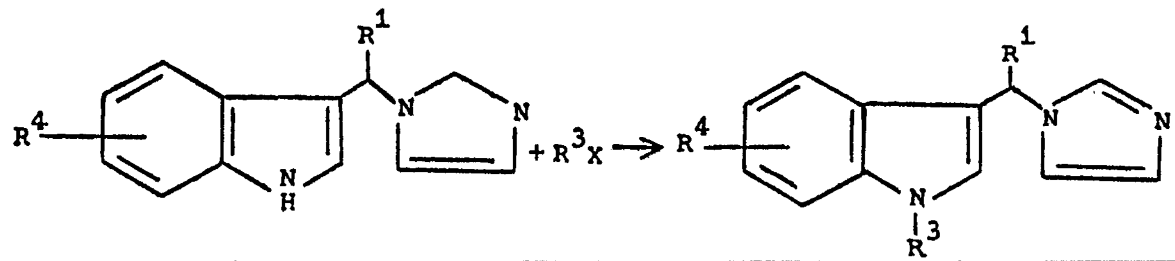

| Example No. | $R^1$ | $R^3$ | $R^4$ | X | Recrystallisation Solvent | m.p. °C | Analysis % (Calculated in brackets) C | H | N |
|---|---|---|---|---|---|---|---|---|---|
| 27 | H | $CH_3$ | H | $OSO_3CH_3$ | Benzene / Petrol (60–80°) | 86–89 (free base) | 73.61 (73.90 | 6.24 6.20 | 19.79 19.89) |
| 28 | $CH_3$ | $CH_3$ | H | I | Ethyl Acetate | 116–118 (fumarate) | 62.67 (63.33 | 5.59 5.61 | 12.01 12.31) |
| 29 | H | $C_2H_5$ | H | $OSO_3C_2H_5$ | Ethyl Acetate/Trace Methanol | 106–107 (maleate) | 63.28 (63.33 | 5.67 5.61 | 12.04 12.31) |
| 30 | H | n-$C_3H_7$ | H | I | Ethyl Acetate / Trace Methanol | 126–127 (fumarate) | 63.92 (64.21 | 5.96 5.96 | 11.84 11.83) |
| 31 | H | $CH_2CH=CH_2$ | H | Br | Ethyl Acetate / Trace Methanol | 120–121 (fumarate) | 64.72 (64.58 | 5.09 5.42 | 12.11 11.89) |
| 32 | $CH_3$ | $CH_2CH=CH_2$ | H | Br | Ethyl Acetate | 113–114 (fumarate) | 65.34 (65 38 | 5.67 5.76 | 11.55 11.44) |
| 33 | H | $CH_2CH=CH_2$ | 5-$OCH_3$ | Br | Ethanol / Ether | 129–130 (oxalate) | 60 2 (60.5 | 5.33 5.35 | 11.81 11.75) |
| 34 | H | $CH_2CH=CH_2$ | 5-Br | Br | Ethanol / Ether | 175–177 (oxalate) | 50 .42 (50.26 | 3.91 3.97 | 10.50 10.34) |

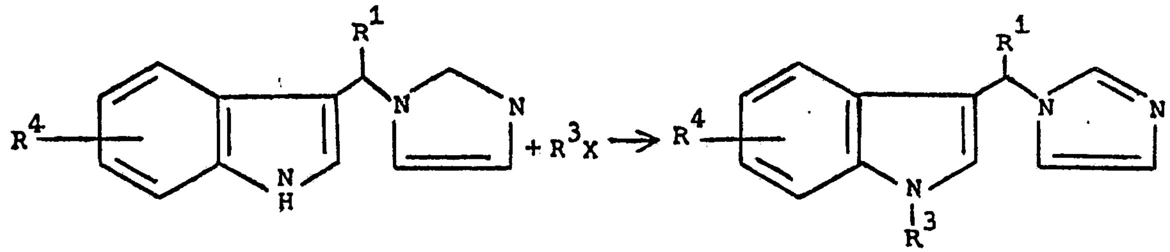

| Example No. | $R^1$ | $R^3$ | $R^4$ | X | Recrystallisation Solvent | m.p. °C | Analysis % (Calculated in brackets) C | H | N |
|---|---|---|---|---|---|---|---|---|---|
| 35 | H | $CH_2CH=CHCH_3$ | H | Br | Ethyl Acetate / Trace Methanol | 141–142 (fumarate) | 65.69 (65.38 | 5.88 5.76 | 11.54 11.44) |
| 36 | H | cyclopropyl-methyl | H | Br | Ethyl Acetate / Trace Methanol | 149–150 (fumarate) | 65.32 (65.38 | 5.71 5.76 | 11.24 11.44) |
| 37 | H | $CH_2CH_2OCH_3$ | H | Br | Ethyl Acetate | 105–107 (fumarate) | 61.17 (61.44 | 5.71 5.70 | 11.09 11.32) |
| 38 | H | $CH_2CH_2N(CH_3)_2$ | H | Cl | Ethyl Acetate / Trace Methanol | 154–156 (bis-oxalate) | 53.17 (53.57 | 5.41 5.39 | 12.38 12.49) |
| 39 | H | 4-chloro-benzyl | H | Cl | Ethyl Acetate / Trace Methanol | 160° (hemi-fumarate) | 66.85 (66.40 | 4.86 4.78 | 11.19 11.06) |
| 40 | H | benzoyl | H | Cl | Ethyl Acetate / Petrol (60–80°) | 121–123 | 75.46 (75.73 | 5.07 5.02 | 13.83 13.95) |
| 41 | H | cyclopropyl-carbonyl | H | Cl | Ethyl Acetate / Petrol (40–60°) | 131–132 | 72.06 (72.43 | 5.82 5.70 | 16.02 15.84) |
| 42 | H | 4-methyl-benzoyl | H | Cl | Toluene | 156 | 76.12 (76.17 | 5.37 5.44 | 13.25 13.32) |

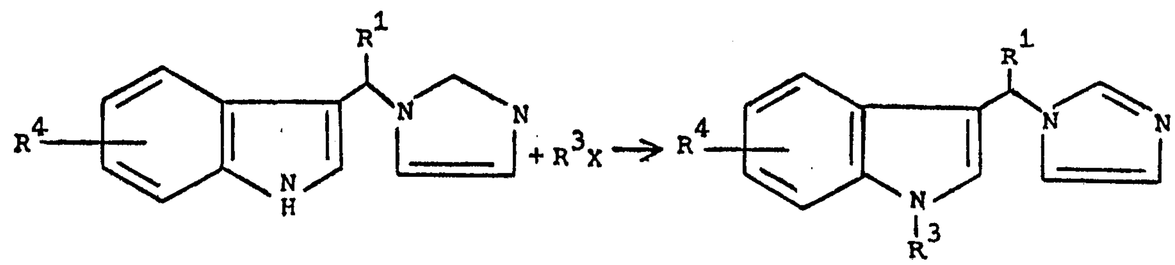

| Example No. | $R^1$ | $R^3$ | $R^4$ | X | Recrystallisation Solvent | m.p. °C | Analysis % (Calculated in brackets) C | H | N |
|---|---|---|---|---|---|---|---|---|---|
| 43 | H | 4-methoxy-benzoyl | H | Cl | Toluene | 132 | 72.10<br>(72.49 | 5.21<br>5.17 | 12.57<br>12.68 |
| 44 | H | 4-chloro-benzoyl | H | Cl | Isopropanol | 174–175<br>(fumarate) | 61.38<br>(61.13 | 4.04<br>4.01 | 9.70<br>9.30) |
| 45 | H | 3-trifluoro-methyl benzoyl | H | Cl | Ethyl Acetate / Trace Isopropanol | 152<br>(fumarate) | 59.28<br>(59.38 | 3.97<br>3.74 | 8.36<br>8.66) |

Example 46

1-Acetyl-3-(imidazol-1-ylmethyl)indole

A mixture of 3-(imidazol-1-ylmethyl)indole (0.97 g) and 1-acetylimidazole (1.10 g) was heated on a steam bath for 3½ hours to give an orange oil which solidified on cooling. The solid was chromatographed on silica gel. Elution with chloroform gave first some impurity followed by pure product. The product-containing fractions were evaporated to give a solid which was crystallised from ethyl acetate to give *1-acetyl-3-(imidazol-1-ylmethyl)indole* (0.50 g), m.p. 122°.

*Analysis %:—*

| | | | |
|---|---|---|---|
| Found: | C, 70.18; | H, 5.43; | N, 17.42 |
| Calculated for $C_{14}H_{13}N_3O$: | C, 70.28; | H, 5.58; | N, 17.56. |

Example 47

5-Hydroxy-3-(imidazol-1-ylmethyl)indole

Boron tribromide (0.38 ml) was added dropwise to a stirred solution of 5-methoxy-3-(imidazol-1-ylmethyl)indole 0.30 g) in dry methylene chloride (30 ml) at −70°. The mixture was allowed to warm up and was stirred at 20° for 5 hours. Water (50 ml) was added and the aqueous layer was separated and washed with methylene chloride (2 x 20 ml). The aqueous layer was basified with solid sodium bicarbonate and then evaporated to dryness.

The residue was boiled with ethyl acetate (200 ml) and the solution was decanted off and evaporated to give a solid. The solid was crystallised from isopropanol/petrol (b.p. 80—100°) to give 5-hydroxy-3-(imidazol-1-ylmethyl)indole (0.10 g), m.p. 169—170°.

*Analysis %:—*

| | | | |
|---|---|---|---|
| Found: | C, 67.59; | H, 5.32; | N, 19.45 |
| Calculated for $C_{12}H_{11}N_3O$: | C, 67.59; | H, 5.20; | N, 19.71. |

Example 48

1-Allyl-5-hydroxy-3-(imidazol-1-ylmethyl)indole

1-Allyl-5-methoxy-3-(imidazol-1-ylmethyl)indole (1.34 g) was treated with boron tribromide (1.91 ml) as described in Example 47. After basification with sodium bicarbonate the aqueous layer was extracted with ethyl acetate (3 x 100 ml). The combined extracts were washed with water and dried ($MgSO_4$). Evaporation of the solvent gave a solid which was crystallised from ethanol to give 1-allyl-5-hydroxy-3-(imidazol-1-ylmethyl)indole (0.56 g), m.p. 165—167°.

*Analysis %:—*

| | | | |
|---|---|---|---|
| Found: | C, 70.72; | H, 6.00; | N, 16.79 |
| Calculated for $C_{15}H_{15}N_3O$: | C, 71.12; | H, 5.97; | N, 16.59. |

Example 49

5-Hydroxy-2-(2-methylphenyl)-3-(imidazol-1-ylmethyl)indole

Treatment of 5-methoxy-2-(2-methylphenyl)-3-(imidazol-1-ylmethyl)indole with boron tribromide as described in Example 47 gave 5-hydroxy-2-(2-methylphenyl)-3-(imidazol-1-ylmethyl)indole, m.p. 217—218° (from ethanol/petrol b.p. 60—80°).

*Analysis %:—*

| | | | |
|---|---|---|---|
| Found: | C, 75.96; | H, 5.99; | N, 13.55 |
| Calculated for $C_{19}H_{17}N_3O$: | C, 75.22; | H, 5.65; | N, 13.85. |

Example 50

2-(4-Hydroxyphenyl)-3-(imidazol-1-ylmethyl)indole

Treatment of 2-(4-methoxyphenyl)-3-(imidazol-1-ylmethyl) indole with boron tribromide and work up as described in Example 47 gave a solid which was chromatographed on silica gel. Elution with chloroform gave a small amount of starting material and the product was eluted with chloroform/methanol (20:1). Evaporation of the product-containing eluate gave a solid which was crystallised from ethanol to give 2-(4-Hydroxyphenyl)-3-(imidazol-1-ylmethyl)indole, m.p. 245—247°.

| | | | |
|---|---|---|---|
| Found: | C, 74.55; | H, 5.35; | N, 14.26 |
| Calculated for $C_{18}H_{15}N_3O$: | C, 74.72; | H, 5.23; | N, 14.52. |

Example 51

3-(Imidazol-1-ylmethyl)-2-*iso*propylindole (250 mg; prepared as in Example 1) was dissolved, with gentle warming to effect solution, in the minimum amount of ethanol (1.5 ml). The solution was then added dropwise to ethereal hydrogen chloride (15 ml) to give a clear solution, which was then allowed to stand for 10 minutes to allow precipitation of the hydrogen chloride salt of 3-(imidazol-1-ylmethyl)-2-*iso*propylindole. The salt was filtered off, recrystallised from ethyl acetate containing a trace of methanol, and dried at 100° for 4 hours (yield 230 mg; m.p. 187—188°).

*Analysis %:—*

| | | | |
|---|---|---|---|
| Calculated for $C_{15}H_{17}N_3{\cdot}HCl$: | C, 65.26; | H, 6.52; | N, 15.23 |
| Found: | C, 64.57; | H, 6.58; | N, 15.57. |

Example 52

3-(Imidazol-1-ylmethyl)indole (1 g) was added to distilled water (900 mls) adjusted to pH 5 with hydrochloric acid. Sodium chloride (18 g) was added and the solution was made up to 2 litres.

This final solution was then sterilised according to the method of the B.P. 1973 by filtration through a suitable bacteria-proof filter under aseptic conditions into sterile containers so as to comply with the test for sterility of Appendix 121, B.P. 1973. Suitable containers are sterile 10 ml glass vials, which are filled with 10 ml of the final solution, i.e. the resulting vials contain 5 mg of the active ingredient.

Example 53

Capsules are compounded from the following ingredients:

| | mg/capsule |
|---|---|
| 3-(Imidazol-1-ylmethyl)indole | 20 |
| Lactose | 250 |
| Maize starch | 75 |
| Magnesium stearate | 5 |
| | 350 mg |

The ingredients are thoroughly blended, granulated and then filled into hard gelatine capsules of the desired size.

Similar solutions and capsules to those described in Examples 52 and 53 are prepared using, instead of 3-(imidazol-1-ylmethyl)indole, a compound of any of Examples 1 to 50 as active ingredient.

The following Example illustrates the preparation of the indole starting material used in Example 1; all temperatures are given in °C:—

Example A

Preparation of 3-(dimethylaminomethyl)-2-*iso*propylindole

2-*Iso*propylindole (9.9 g) in ethanol (30 ml) was stirred at 0° and to this solution was added 33% (wt./wt.) dimethylamine in ethanol (10 ml) and glacial acetic acid (3.5 ml). 40% (wt./vol.) Aqueous formaldehyde (5 ml) was then added dropwise, and the solution stirred at 5° for 1 hour.

Ice/water was added, followed by excess solid potassium carbonate to give an orange oil. The solution was then extracted with ether (3 x 50 ml). The combined ethereal extracts were dried ($Na_2SO_4$) and evaporated to give crude 3-(dimethylaminomethyl)-2-*iso*propylindole (2-*iso*propyl-gramine) (4.5 g) as a brown oil which was used directly in Example 1 without further purification.

Other 3-(dimethylaminomethyl)-indoles used as starting materials for Examples 2 to 6 and 8 to 25 are either known compounds or are prepared from the appropriately substituted indoles by methods similar to the above. The starting material for Example 7 is described in "Tetrahedron", 1973, *29,* 3357.

Compounds of the invention have been tested by the *in vitro* enzyme assays hereinbefore described and the results of the these tests are shown in the following Table, which gives the molar concentration of each compound which caused a 50% change in the effect of the relevant enzyme on isometric tension, i.e. caused a 50% inhibition of the action of that enzyme. Also shown are the ratios of molar concentrations causing 50% inhibition of the actions of the prostacyclin synthetase and thromboxane synthetase enzymes, which gives an indication of the ability of compounds to selectively inhibit the action of the latter enzyme relative to the former.

| Example No. | Concentration causing 50% inhibition of | | | Ratio of (3) to (1) |
|---|---|---|---|---|
| | (1) thromboxane synthetase | (2) cyclo-oxygenase | (3) prostacyclin synthetase | |
| * | $3 \times 10^{-8}$ | $>10^{-3}$ | $6.5 \times 10^{-4}$ | 22,000 |
| 1 | $1.7 \times 10^{-8}$ | $>10^{-4}$ | $>10^{-4}$ | >6,000 |
| 2 | $1.2 \times 10^{-8}$ | $>10^{-4}$ | $3 \times 10^{-5}$ | 2,500 |
| 3 | $1.0 \times 10^{-8}$ | $>10^{-4}$ | $1.2 \times 10^{-5}$ | 1,200 |
| 4 | $2 \times 10^{-7}$ | $>10^{-4}$ | $1 \times 10^{-4}$ | 500 |
| 5 | $6.2 \times 10^{-8}$ | $>10^{-4}$ | $4 \times 10^{-6}$ | 65 |
| 6 | $3 \times 10^{-9}$ | $>10^{-4}$ | $9.5 \times 10^{-7}$ | 32 |
| 7 | $3 \times 10^{-8}$ | | $>10^{-4}$ | 3,300 |
| 8 | | | | |
| 9 | $1.0 \times 10^{-7}$ | $>10^{-4}$ | $1.7 \times 10^{-5}$ | 170 |
| 10 | $8.4 \times 10^{-9}$ | $>10^{-4}$ | $>10^{-4}$ | >12,000 |
| 11 | $5.8 \times 10^{-8}$ | | $3.5 \times 10^{-5}$ | 600 |

*Compound in which $R^1$, $R^2$, $R^3$ and $R^4$ are each hydrogen.

| Example No. | Concentration causing 50% inhibition of | | | Ratio of (3) to (1) |
|---|---|---|---|---|
| | (1) thromboxane synthetase | (2) cyclo-oxygenase | (3) prostacyclin synthatase | |
| 12 | $3 \times 10^{-10}$ | $>10^{-4}$ | $2.1 \times 10^{-5}$ | 70,000 |
| 13 | $1 \times 10^{-10}$ | $>10^{-4}$ | $8.4 \times 10^{-7}$ | 8,400 |
| 14 | $2.5 \times 10^{-9}$ | | $1 \times 10^{-6}$ | 400 |
| 15 | $1.4 \times 10^{-11}$ | | $1.4 \times 10^{-5}$ | $10^{6}$ |
| 16 | | | | |
| 17 | $2.5 \times 10^{-10}$ | $>10^{-4}$ | $2 \times 10^{-6}$ | 8,000 |
| 18 | $3.8 \times 10^{-10}$ | | $1.4 \times 10^{-6}$ | 3,700 |
| 19 | $1.2 \times 10^{-7}$ | | | |
| 20 | $3 \times 10^{-9}$ | $>10^{-4}$ | $4 \times 10^{-5}$ | 13,000 |
| 21 | $1.1 \times 10^{-7}$ | | $>10^{-4}$ | >900 |
| 22 | $1 \times 10^{-9}$ | | $>10^{-4}$ | $>10^{5}$ |
| 23 | $6.4 \times 10^{-8}$ | | $>10^{-4}$ | >1,500 |

| Example No. | Concentration causing 50% inhibition of | | | Ratio of (3) to (1) |
|---|---|---|---|---|
| | (1) thromboxane synthetase | (2) cyclo-oxygenase | (3) prostacyclin synthetase | |
| 24 | $2 \times 10^{-7}$ | | $1 \times 10^{-4}$ | 500 |
| 25 | $2.6 \times 10^{-7}$ | | $3.7 \times 10^{-5}$ | 140 |
| 26 | $6.5 \times 10^{-8}$ | $>10^{-4}$ | $1 \times 10^{-6}$ | 15 |
| 27 | $2 \times 10^{-9}$ | $>10^{-4}$ | $>10^{-4}$ | >50,000 |
| 28 | $3.3 \times 10^{-8}$ | | | |
| 29 | $1.4 \times 10^{-9}$ | | $>10^{-4}$ | >70,000 |
| 30 | $4 \times 10^{-9}$ | | $>10^{-4}$ | |
| 31 | $5.8 \times 10^{-11}$ | | $>10^{-4}$ | $1.7 \times 10^{-6}$ |
| 32 | $3.6 \times 10^{-8}$ | | | |
| 33 | | | | |
| 34 | | | | |
| 35 | $3.3 \times 10^{-9}$ | | $1 \times 10^{-6}$ | 300 |

| Example No. | Concentration causing 50% inhibition of | | | Ratio of (3) to (1) |
|---|---|---|---|---|
| | (1) thrombozane synthetase | (2) cyclo-oxygenase | (3) prostacyclin synthetase | |
| 36 | $1.1 \times 10^{-8}$ | | $1 \times 10^{-4}$ | 9,000 |
| 37 | $4.5 \times 10^{-8}$ | | $>10^{-4}$ | >2,200 |
| 38 | $2.6 \times 10^{-8}$ | | $>10^{-4}$ | >3,800 |
| 39 | $8.6 \times 10^{-8}$ | | $1.8 \times 10^{-5}$ | 210 |
| 40 | $2 \times 10^{-9}$ | $>10^{-4}$ | $1.7 \times 10^{-6}$ | 850 |
| 41 | $4.3 \times 10^{-9}$ | | | |
| 42 | $4.1 \times 10^{-10}$ | | $2 \times 10^{-5}$ | 49,000 |
| 43 | $1.7 \times 10^{-9}$ | | $>10^{-4}$ | >60,000 |
| 44 | | | | |
| 45 | $1.0 \times 10^{-7}$ | | | |
| 46 | $1.2 \times 10^{-9}$ | | $6.8 \times 10^{-5}$ | 57,000 |
| 47 | $2.3 \times 10^{-9}$ | | $8 \times 10^{-6}$ | 3,500 |

| Example No. | Concentration causing 50% inhibition of | | | Ratio of (3) to (1) |
|---|---|---|---|---|
| | (1) thromboxane synthetase | (2) cyclo-oxygenase | (3) prostacyclin synthetase | |
| 48 | | | | |
| 49 | | | | |
| 50 | $6.6 \times 10^{-9}$ | $>10^{-4}$ | $3 \times 10^{-6}$ | 450 |

The results given in the Table show that all of the compounds tested caused a 50% inhibition of the thromboxane synthetase enzyme at a molar concentration of $2.6 \times 10^{-7}$ or less, and several caused 50% inhibition at concentrations of $10^{-9}$ or less.

Of the compounds tested for inhibition of the cyclooxygenase enzyme, none caused 50% inhibition at a molar concentration of $10^{-4}$ or less, their ability to inhibit that enzyme being at least 2,600 times less, and in several cases 10,000 times less, than their ability to inhibit the thromboxane synthetase enzyme.

Of the compounds tested for inhibition of the prostacyclin synthetase enzyme, none caused 50% inhibition at a molar concentration less than 15 times greater than that at which they caused 50% inhibition of the thromboxane synthetase enzyme, i.e. they were all at least 15 times more potent as inhibitors of thromboxane synthetase than of prostacyclin synthetase. Most of the compounds were at least 100 times, while many were 1000 times and some even 1000000 times more potent.

It is expected that all the compounds of the invention when tested in this way will give results within the range of those already tested.

**Claims**

1. A compound of the formula:

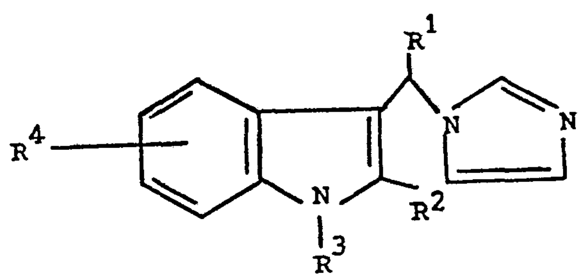

(I)

wherein

$R^1$ is hydrogen or lower alkyl;

$R^2$ is hydrogen, lower alkyl, lower cycloalkyl, adamantyl, or a phenyl or benzyl group in which the benzene ring is optionally mono-substituted with halogen, hydroxy, lower alkyl, lower alkoxy or trifluoromethyl;

$R^3$ is hydrogen, lower alkyl, allyl, 3-methyl-allyl, lower cycloalkyl-methyl, lower cycloalkyl-ethyl, lower alkoxy-lower alkyl, di(lower alkyl)amino-lower alkyl, lower alkanoyl, lower cycloalkylcarbonyl, or a benzyl or benzoyl group in which the benzene ring is optionally substituted as in $R^2$;

$R^4$ is hydrogen, lower alkyl, lower alkoxy, halogen, hydroxy, trifluoromethyl, di(lower alkyl)amino, or a benzyloxy group in which the benzene ring is optionally substituted as in $R^2$;

lower when applied to an alkyl, alkoxy or alkanoyl group means that the group contains not more than 4 carbon atoms, and when applied to a cycloalkyl group means that the group contains not more than 7 carbon atoms; *with the proviso* that at least one of $R^1$, $R^2$, $R^3$ and $R^4$ is other than hydrogen; and the pharmaceutically acceptable acid addition salts thereof.

2. A compound according to claim 1 but in which $R^1$, $R^2$, $R^3$ and $R^4$ are hydrogen, or a pharmaceutically acceptable acid addition salt thereof, for use in treating an animal, including a human being, to inhibit the action of the thromboxane synthetase enzyme without significantly inhibiting the action of the prostacyclin synthetase or cyclo-oxygenase enzymes.

3. A compound according to claim 1, in which $R^1$ is hydrogen or methyl.

4. A compound according to claim 1 or 3, in which $R^3$ is hydrogen and $R^2$ is hydrogen, isopropyl, cyclopropyl, cyclohexyl, benzyl, o- or p-tolyl or o-chloro-phenyl.

5. A compound according to claim 1 or 3, in which $R^2$ is hydrogen and $R^3$ is methyl, ethyl, n-propyl, allyl, cyclopropylmethyl acetyl or p-methyl- or p-methoxy-benzoyl.

6. A compound according to any of claims 1 or 3 to 5, in which $R^4$ is hydrogen.

7. A pharmaceutical composition comprising a compound according to claim 1, or a pharmaceutically acceptable acid addition salt thereof, together with a pharmaceutically acceptable diluent or carrier.

8. A pharmaceutical composition in unit dosage form comprising a compound according to any of claims 2 to 6, or a pharmaceutically acceptable acid addition salt thereof, together with a pharmaceutically acceptable diluent or carrier.

9. A method of preparing a compound according to claim 1, in which $R^3$ is hydrogen, which comprises reacting an indole of the formula:

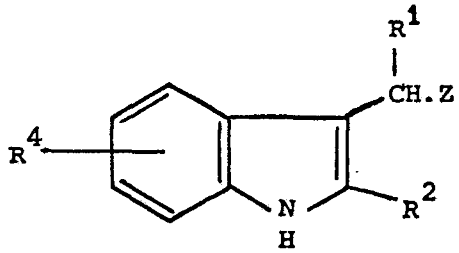

(II)

wherein $R^1$, $R^2$ and $R^4$ are as defined in claim 1 and Z is $-N(C_1-C_4 \text{ alkyl})_2$, $-\overset{\oplus}{N}(C_1-C_4 \text{ alkyl})_3$, —Cl, —Br, $-OSO_2(C_1-C_4 \text{ alkyl})$ or $-OSO_2$ (phenyl, tolyl or *p*-methoxyphenyl), with imidazole.

10. A method of preparing a compound according to claim 1, in which $R^3$ is other than hydrogen, which comprises reacting the corresponding compound in which $R^3$ is hydrogen with an appropriate alkylating or acylating agent in the presence of a base which forms the corresponding N-anion of the starting compound.

**Revendications**

1. Un composé de formule:

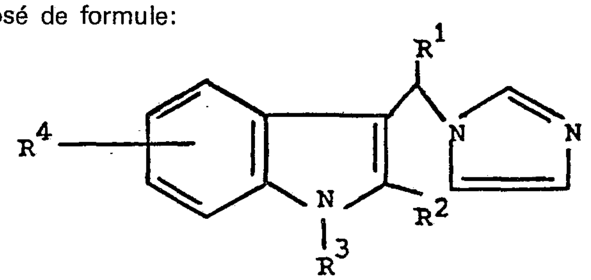

(I)

dans laquelle:

$R^1$ est l'hydrogène ou un groupe alkyle inférieur;

$R^2$ est l'hydrogène, un groupe alkyle inférieur, cycloalkyle inférieur, adamantyle ou un groupe phényle ou benzyle dont le noyau benzénique est éventuellement mono-substitué par un halogène ou un radical hydroxy, alkyle inférieur, alkoxy inférieur ou trifluorométhyle;

$R^3$ est l'hydrogène, un groupe alkyle inférieur, allyle, 3-méthylallyle, (cycloalkyle inférieur)-méthyle, (cycloalkyle inférieur)-éthyle, (alkoxy inférieur)-alkyle inférieur, di-(alkyle inférieur)-amino-alkyle inférieur, alcanoyle inférieur, (cycloalkyle inférieur)-carbonyle ou un groupe benzyle ou benzoyle dont le noyau benzénique est éventuellement substitué comme dans $R^2$;

et $R^4$ est l'hydrogène ou un groupe alkyle inférieur, alkoxy inférieur, un halogène, un groupe hydroxy, trifluorométhyle, di-(alkyle inférieur)-amino, ou un groupe benzyloxy dont le noyau benzénique est éventuellement substitué comme dans $R^2$;

le terme inférieur appliqué à un groupe alkyle, alkoxy ou alcanoyle signifiant que le groupe ne contient pas plus de 4 atomes de carbone et ce terme appliqué à un groupe cycloalkyle signifiant que le groupe ne contient pas plus de 7 atomes de carbone; *à condition* qu'au moins l'un des symboles $R^1$, $R^2$, $R^3$ et $R^4$ désigne autre chose que de l'hydrogène; et leurs sels d'addition d'acides acceptables du point de vue pharmaceutique.

2. Composé suivant la revendication 1, dans lequel $R^1$, $R^2$, $R^3$ et $R^4$ représentent de l'hydrogène ou un sel d'addition d'acide acceptable du point de vue pharmaceutique de ce composé, destiné à être utilisé dans le traitement d'un animal, ainsi que d'un être humain, pour inhiber l'action de l'enzyme appelé thromboxane-synthétase sans inhiber notablement l'action des enzymes appelés prostacycline-synthétase ou cyclo-oxygénase.

3. Composé suivant la revendication 1, dans lequel $R^1$ est l'hydrogène ou le groupe méthyle.

4. Composé suivant la revendication 1 ou 3, dans lequel $R^3$ est l'hydrogène et $R^2$ est l'hydrogène ou un groupe isopropyle, cyclopropyle, cyclohexyle, benzyle, o- ou p-tolyle ou o-chlorophényle.

5. Composé suivant la revendication 1 à 3, dans lequel $R^2$ est l'hydrogène et $R^3$ est un groupe méthyle, éthyle, n-propyle, allyle, acétyle ou p-méthyl- ou p-méthoxy-benzoyle.

6. Composé suivant l'une quelconque des revendications 1 ou 3 à 5, dans lequel $R^4$ est l'hydrogène.

7. Composition pharmaceutique, comprenant un composé suivant la revendication 1 ou un sel d'addition d'acide acceptable du point de vue pharmaceutique de ce composé, en association avec un diluant ou support acceptable du point de vue pharmaceutique.

8. Composition pharmaceutique sous la forme posologique unitaire, comprenant un composé suivant l'une quelconque des revendications 2 à 6 ou un sel d'addition d'acide acceptable du point de vue pharmaceutique de ce composé, en association avec un diluant ou support acceptable du point de vue pharmaceutique.

9. Procédé de préparation d'un composé suivant la revendication 1, dans lequel $R^3$ est l'hydrogène, qui consiste à faire réagir un indole de formule:

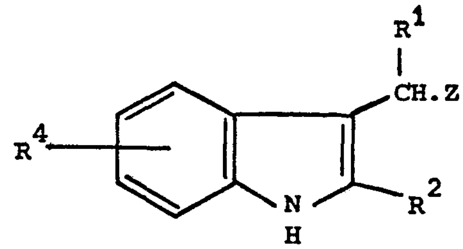

(II)

dans laquelle $R^1$, $R^2$ et $R^4$ ont la définition donnée dans la revendication 1 et Z est un groupe —N(alkyle en $C_1$ à $C_4$)$_2$, —$\overset{\oplus}{N}$(alkyle en $C_1$ à $C_4$)$_3$, —Cl, —Br, —$OSO_2$(alkyle en $C_1$ à $C_4$) ou —$OSO_2$ (phényle, tolyle ou p-méthoxyphényle), avec l'imidazole.

10. Procédé de préparation d'un composé suivant la revendication 1, dans lequel $R^3$ est autre chose que l'hydrogène, qui consiste à faire réagir le composé correspondant dans lequel $R^3$ est l'hydrogène avec un agent alkylant ou acylant convenable en présence d'une base qui forme le N-anion correspondant du composé de départ.

**Patentansprüche**

1. Verbindung der Formel

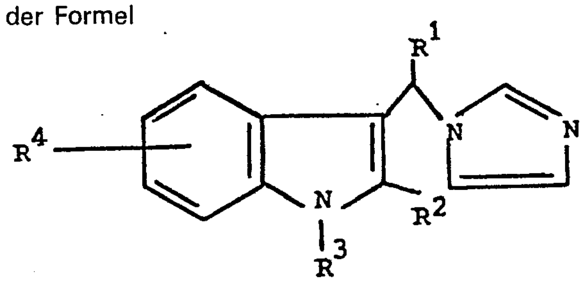

(I)

worin

$R^1$ Wasserstoff oder nieder-Alkyl ist;

$R^2$ Wasserstoff, nieder-Alkyl, nieder-Cycloalkyl, Adamantyl oder eine Phenyl- oder Benzyl-Gruppe, in welcher der Benzolring gegebenenfalls mit Halogen, Hydroxy, nieder-Alkyl, nieder-Alkoxy oder Trifluoromethyl mono-substituiert ist, bedeutet;

$R^3$ Wasserstoff, nieder-Alkyl, Allyl, 3-Methyl-allyl, nieder-Cycloalkyl-methyl, nieder-Cycloalkyl-äthyl, nieder-Alkoxy-nieder-Alkyl, Di(nieder-alkyl)amino-nieder-alkyl, nieder-Alkanoyl, nieder-Cycloalkyl-carbonyl oder eine Benzyl- oder Benzoyl-Gruppe, in welcher der Benzolring gegebenenfalls wie in $R^2$ substituiert ist, bedeutet;

$R^4$ Wasserstoff, nieder-Alkyl, nieder-Alkoxy, Halogen, Hydroxy, Trifluoromethyl, Di(nieder-alkyl)-amino oder eine Benzyloxy-Gruppe, in welcher der Benzolring gegebenenfalls wie in $R^2$ substituiert ist, bedeutet;

nieder, wenn angewandt auf eine Alkyl-, Alkoxy- oder Alkanoyl-Gruppe bedeutet, daß die Gruppe nicht mehr als 4 Kohlenstoffatome enthält und wenn auf eine Cycloalkyl-Gruppe angewendet bedeutet, daß die Gruppe nicht mehr als 7 Kohlenstoffatome enthält; mit dem Vorbehalt, daß mindestens einer der Substituenten $R^1$, $R^2$, $R^3$ und $R^4$ etwas anderes bedeutet als Wasserstoff; und die pharmazeutisch annehmbaren Säureadditionssalze davon.

2. Verbindung gemäß Anspruch 1, in welcher jedoch $R^1$, $R^2$, $R^3$ und $R^4$ Wasserstoff bedeuten oder ein pharmazeutisch annehmbares Säureadditionssalz davon, zur Verwendung bei der Behandlung eines Tieres, einschließlich des Menschen, zwecks Inhibierung der Wirkung des Thromboxan-Synthetaseenzyms ohne wesentliche Inhibierung der Wirkung der Prostacyclin-Synthetase oder Cyclo-Oxygenaseenzyme.

3. Verbindung gemäß Anspruch 1, worin $R^1$ Wasserstoff oder Methyl bedeutet.

4. Verbindung gemäß Anspruch 1 oder 3, worin $R^3$ Wasserstoff und $R^2$ Wasserstoff, Isopropyl, Cyclopropyl, Cyclohexyl, Benzyl, o- oder p-Tolyl oder o-Chloro-phenyl bedeutet.

5. Verbindung gemäß Anspruch 1 oder 3, worin $R^2$ Wasserstoff bedeutet und $R^3$ Methyl, Äthyl, n-Propyl, Allyl, Acetyl oder p-Methyl- oder p-Methoxy-benzoyl bedeutet.

6. Verbindung gemäß einem der Ansprüche 1 oder 3 bis 5, worin $R^4$ Wasserstoff bedeutet.

7. Pharmazeutische Zusammensetzung enthaltend eine Verbindung gemäß Anspruch 1 oder ein pharmazeutisch annehmbares Säureadditionssalz davon zusammen mit einem pharmazeutisch annehmbaren Verdünnungsmittel oder Träger.

8. Pharmazeutische Zusammensetzung in Form einer Dosierungseinheit enthaltend eine Verbindung gemäß einem der Ansprüche 2 bis 6 oder ein pharmazeutisch annehmbares Säureadditionssalz davon, zusammen mit einem pharmazeutisch annehmbaren Verdünnungsmittel oder Träger.

9. Verfahren zur Herstellung einer Verbindung gemäß Anspruch 1, worin $R^3$ Wasserstoff bedeutet, wobei ein Indol der Formel

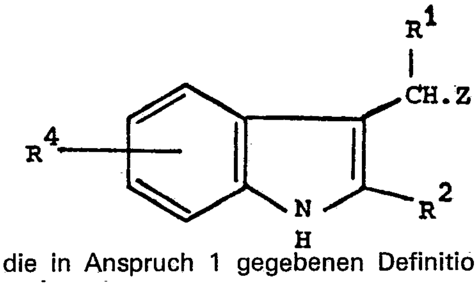

(II)

worin $R^1$, $R^2$ und $R^4$ die in Anspruch 1 gegebenen Definitionen besitzen und Z $—N(C_1—C_4alkyl)_2$, $—\overset{\oplus}{N}(C_1—C_4alkyl)_3$, —Cl, —Br, $OSO_2(C_1—C_4alkyl)$ oder $—OSO_2$(phenyl, tolyl oder p-methoxy-phenyl) bedeutet mit Imidazol umgesetzt wird.

10. Verfahren zur Herstellung einer Verbindung gemäß Anspruch 1, worin $R^3$ von Wasserstoff unterschiedlich ist, wobei die entsprechende Verbindung, in welcher $R^3$ Wasserstoff bedeutet, mit einem geeigneten Alkylierungs- oder Acylierungsmittel in Anwesenheit einer Base, die das entsprechende N-Anion der Ausgangsverbindung bildet, umgesetzt wird.